# EUROPÄISCHE PATENTSCHRIFT

(11) **EP 1 299 073 B1**
(45) Veröffentlichungstag und Bekanntmachung des Hinweises auf die Patenterteilung: **20.04.2005**
(21) Anmeldenummer: 01960334.9
(22) Anmeldetag: 20.06.2001
(51) Int. Cl.: A61K 7/13, C07D 307/00, C07D 493/00

(54) **MITTEL ZUM FÄRBEN KERATINISCHER FASERN, DIE DERIVATE DES BENZO[B]FURAN-3-ONS UND/ODER BENZO[B]THIOPHEN-3-ONS ENTHALTEN**
AGENTS FOR COLOURING KERATIN FIBRES, CONTAINING DERIVATIVES OF BENZO(B)FURANE-3-ONE AND/OR BENZO(B)THIOPHENE-3-ONE
AGENTS POUR LA COLORATION DES FIBRES KERATINIQUES CONTENANT DES DERIVES DE BENZO(B)FURANE-3-ONE ET/OU DE BENZO(B)THIOPHENE-3-ONE.

(30) Priorität: 29.06.2000 DE 10030646
(43) Veröffentlichungstag der Anmeldung: 09.04.2003
(73) Patentinhaber: Henkel Kommanditgesellschaft auf Aktien, 40589 Düsseldorf (DE)
(72) Erfinder: HÖFFKES, Horst, 40595 Düsseldorf (DE); SCHUMANN, Klaus, 40699 Erkrath (DE); HOLLENBERG, Detlef, 40699 Erkrath (DE); DÜMELAND, Martin, 38120 Braunschweig (DE); GRAHN, Walter, 38110 Braunschweig (DE)
(86) Internationale Anmeldenummer: PCT/EP2001/006929
(87) Internationale Veröffentlichungsnummer: WO 2002/000180

(56) Entgegenhaltungen:
- EP-A- 0 380 223
- EP-A- 1 133 978
- WO-A-01/55128
- WO-A-89/05289
- WO-A-98/09956
- DE-A- 2 936 730
- PATENT ABSTRACTS OF JAPAN vol. 005, no. 046 (P-054), 27. März 1981 (1981-03-27) & JP 56 001047 A (ORIENTAL SHASHIN KOGYO KK), 8. Januar 1981 (1981-01-08)

## Beschreibung

Die vorliegende Erfindung betrifft Mittel zum Färben keratinischer Fasern, die Derivate des Benzo[b]furan-3-ons und/oder des Benzo[b]thiophen-3-ons enthalten, ein Färbeverfahren mit diesen Farbstoffvorprodukten, die Verwendung der Derivate des Benzo[b]furan-3-ons und/oder des Benzo[b]thiophen-3-ons zum Färben keratinischer Fasern sowie neue Derivate des Benzo[b]furan-3-ons und des Benzo[b]thiophen-3-ons.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel empfindlich, so daß eine vielfach unerwünschte Nuancenverschiebung oder gar eine sichtbare "Entfärbung" eintreten kann.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muß üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Schließlich hat in jüngster Zeit ein neuartiges Färbeverfahren große Beachtung gefunden. Bei diesem Verfahren werden Vorstufen des natürlichen Haarfarbstoffes Melanin auf das Haar aufbracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B1-530 229 beschrieben.

Das Patentdokument WO 01/55128 A offenbart die Verwendung von Benzofuranonderivaten in Haarfärbemitteln.

Dennoch wurde stets nach weiteren Färbesystemen gesucht, die eine intensive Färbung der Fasern mit ausgezeichneten Echtheitseigenschaften ermöglichen.

Es wurde nunmehr überraschenderweise gefunden, daß sich spezielle Derivate des Benzo[b]furan-3-ons und/oder des Benzo[b]thiophen-3-ons hervorragend als Farbstoffvorprodukte zum Färben keratinischer Fasern eignen.

Ein erster Gegenstand der vorliegenden Erfindung ist daher ein Mittel zur Färbung keratinischer Fasern, enthaltend in einem kosmetisch akzeptablen Medium als Farbstoffvorprodukt mindestens ein spezielles Derivat des Benzo[b]furan-3-ons und/oder mindestens ein spezielles Derivat des Benzo[b]thiophen-3-ons und/oder deren Keto-Enol-Tautomere.

Unter keratinischen Fasern werden erfindungsgemäß Pelze, Wolle, Federn und insbesondere menschliche Haare verstanden.

Die speziellen Derivate des Benzo[b]furan-3-ons und des Benzo[b]thiophen-3-ons im Sinne der vorliegenden Erfindung sind die Derivate der Formel (1) 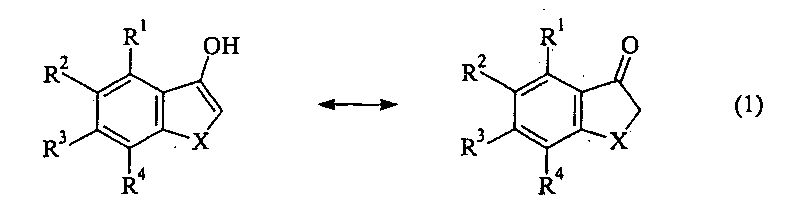 worin
X steht für Schwefel oder Sauerstoff und
die Reste R¹, R², R³ und R⁴ stehen unabhängig voneinander für Wasserstoff,
- eine C₁₋₄-Alkylgruppe, die gegebenenfalls mit
   - einer oder mehreren Hydroxygruppe(n)
   - einer gegebenenfalls mit einer C₁₋₄-Alkylgruppe veresterten Carboxygruppe oder
   - einer Gruppe -SO₃R⁵, wobei R⁵ steht für Wasserstoff oder eine C₁₋₄-Alkylgruppe
   substituiert sein kann,
- eine gegebenenfalls substituierte Amino- C₁₋₄-alkylgruppe,
- eine gegebenenfalls substituierte Amino-C₁₋₄-alkylaminogruppe,
- eine perfluorierte C₁₋₄-Alkylgruppe,
- eine Cyanogruppe,
- eine Allylgruppe,
- eine Vinylgruppe,
- ein Halogenatom,
- eine Gruppe -SO₃R⁵ oder
- eine Gruppe -OR⁶, wobei R⁶ steht für
   - Wasserstoff,
   - eine C₁₋₄-Alkylgruppe, die gegebenenfalls mit
      - einer oder mehreren Hydroxygruppe(n),
      - einer gegebenenfalls mit einer C₁₋₄-Alkylgruppe veresterten Carboxygruppe oder
      - einer Gruppe -SO₃R⁵
      substituiert sein kann,
   - eine perfluorierte C₁₋₄-Alkylgruppe.

Beispiele für die als Substituenten in den erfindungsgemäßen Verbindungen genannten C₁₋₄-Alkylgruppen sind die Gruppen Methyl, Ethyl, Propyl, Isopropyl und Butyl. Ethyl und Methyl sind bevorzugte Alkylgruppen. Bevorzugte C₁₋₄-Hydroxyalkylgruppen sind die Gruppen Hydroxymethyl, 2-Hydroxyethyl, 3-Hydroxypropyl oder 4-Hydroxybutyl; 2-Hydroxyethyl ist eine besonders bevorzugte Hydroxyalkylgruppe. Die weiteren verwendeten Begriffe leiten sich erfindungsgemäß von den hier gegebenen Definitionen ab Eine erfindungsgemäß bevorzugte perfluorierte C₁₋₄-Alkylgruppe ist die Gruppe TrifluormetHyl. Eine erfindungsgemäß bevorzugt Carboxy-C₁₋₄-alkylgruppe ist die Gruppe Carboxymethyl. Beispiele für ein Halogenatom sind erfindungsgemäß ein F-, ein Cl- oder ein Br-Atom, ein Cl-Atom ist besonders bevorzugt. Bevorzugte Amino-C₁₋₄-alkylgruppen sind die Gruppen Aminomethyl, Aminoethyl, Diethylaminomethyl und Dimethylaminomethyl. Eine bevorzugte Amino-C₁₋₄-alkylaminogruppe ist die Aminoethylaminogruppe.

Da es sich bei den Verbindungen der Formel (1) um Substanzen handelt, die in einem Keto-Enol-Gleichgewicht vorliegen, beziehen sich alle Aussagen der vorliegenden Anmeldung sowohl auf die Keto-Form dieser Verbindungen als auch auf die Enol-Form.

Als besonders vorteilhaft haben sich die Verbindungen der Formel (1) erwiesen, bei denen R¹ und/oder R⁴ für Wasserstoff stehen.

Weiterhin können Verbindungen der Formel (1) bevorzugt sein, bei denen R² für einen Substituenten der Formel -OR⁶ steht.

Besonders bevorzugt ist R² ausgewählt aus einer Hydroxygruppe, einer Methoxygruppe, einer Carboxymethylgruppe und einer 2-Hydroxyethoxygruppe.

In einer ersten Variante der vorliegenden Erfindung sind Verbindungen der Formel (1) bevorzugt, bei denen X für ein Sauerstoffatom steht, wie beispielsweise die Verbindungen 5-Carboxymethoxy-benzo[b]furan-3-on, 5-Hydroxy-benzo[b]furan-3-on oder 5-(2'-Hydroxyethoxy)-benzo[b]furan-3-on.

In einer zweiten Variante der vorliegenden Erfindung sind Verbindungen bevorzugt, bei denen X für ein Schwefelatom steht, wie beispielsweise die Verbindungen 5-Hydroxy-benzo[b]thiophen-3-on, 5-Carboxymethoxy-benzo[b]thiophen-3-on, 5,6-Dihydroxy-benzo[b]thiophen-3-on oder 6-Hydroxy-5-methoxy-benzo[b]thiophen-3-on.

In einer bevorzugten Ausführungsform der vorliegenden Erfindung werden die Benzo[b]furan-3-on- und/oder Benzo[b]thiophen-3-on-Derivate als einzige Farbstoffvorprodukte eingesetzt.

In einer weiteren Ausführungsform können die erfindungsgemäßen farbverändemden Mittel aber auch noch weitere Farbstoffe und/oder Farbstoffvorprodukte enthalten.

Hinsichtlich der in den erfindungsgemäßen Mitteln einsetzbaren Farbstoffvorprodukten unterliegt die vorliegende Erfindung keinerlei Einschränkungen. Die erfindungsgemäßen Mittel können als Farbstoffvorprodukte
- Oxidationsfarbstoffvorprodukte vom Entwickler- und Kuppler-Typ, und
- Vorstufen naturanaloger Farbstoffe, wie Indol- und Indolin-Derivate,
sowie Mischungen von Vertretern dieser Gruppen enthalten.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (2) 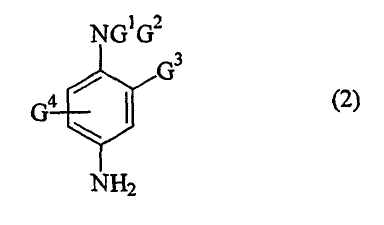 wobei
- G¹ steht für ein Wasserstoffatom, ein C₁- bis C₄-Alkylradikal, ein C₁- bis C₄-Monohydroxyalkylradikal, ein C₂- bis C₄-Polyhydroxyalkylradikal, ein (C₁- bis C₄)-Alkoxy-(C₁- bis C₄)-alkylradikal, ein 4'-Aminophenylradikal oder ein C₁- bis C₄-Alkylradikal, das mit einer stickstoffhaltigen Gruppe, einem Phenyl- oder einem 4'-Aminophenylrest substituiert ist;
- G² steht für ein Wasserstoffatom, ein C₁- bis C₄-Alkylradikal, ein C₁- bis C₄-Monohydroxyalkylradikal, ein C₂- bis C₄-Polyhydroxyalkylradikal, ein (C₁- bis C₄)-Alkoxy-(C₁- bis C₄)-alkylradikal oder ein C₁- bis C₄-Alkylradikal, das mit einer stickstoffhaltigen Gruppe substituiert ist;
- G³ steht für ein Wasserstoffatom, ein Halogenatom, wie ein Chlor-, Brom, Jod- oder Fluoratom, ein C₁- bis C₄-Alkylradikal, ein C₁- bis C₄-Monohydroxyalkylradikal, ein C₁- bis C₄-Hydroxyalkoxyradikal, ein C₁- bis C₄-Acetylaminoalkoxyradikal, ein C₁- bis C₄-Mesylaminoalkoxyradikal oder ein C₁- bis C₄-Carbamoylaminoalkoxyradikal;
- G⁴ steht für ein Wasserstoffatom, ein Halogenatom oder ein C₁- bis C₄-Alkylradikal oder
- wenn G³ und G⁴ in ortho-Stellung zueinander stehen, können sie gemeinsam eine verbrückende α,ω-Alkylendioxogruppe, wie beispielsweise einen Ethylendioxygruppe bilden.

Beispiele für die als Substituenten in den erfindungsgemäßen Verbindungen genannten, C₁- bis C₄-Alkylradikale sind die Gruppen Methyl, Ethyl, Propyl, Isopropyl und Butyl. Ethyl und Methyl sind bevorzugte Alkylradikale. Erfindungsgemäß bevorzugte C₁- bis C₄-Alkoxyradikale sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyalkylgruppe ist besonders bevorzugt. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (2) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (2) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-bis-(β-Hydroxyethyl)-p-phenylendiamin, 4-N,N-bis-(β-Hydroxyethyl)amino-2-methylanilin, 4-N,N-bis-(β-Hydroxyethyl)amino-2-chloranilin, 2-(β-Hydroxyethyl)-p-phenylendiamin, 2-Fluor-pphenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,-β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (2) sind p-Phenylendiamin, p-Toluylendiamin, 2-(β-Hydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (3) entsprechen, sowie ihre physiologisch verträglichen Salze: 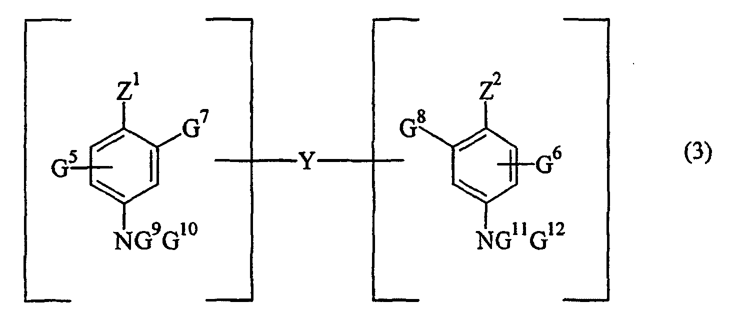 wobei:
- Z¹ und Z² stehen unabhängig voneinander für ein Hydroxyl- oder NH₂-Radikal, das gegebenenfalls durch ein C₁- bis C₄-Alkylradikal, durch ein C₁- bis C₄-Hydroxyalkylradikal und/oder durch eine Verbrückung Y substituiert ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch ein oder mehrere Hydroxyl- oder C₁- bis C₈-Alkoxyradikale substituiert sein kann,
- G⁵ und G⁶ stehen unabhängig voneinander für ein Wasserstoff- oder Halogenatom, ein C₁- bis C₄-Alkylradikal, ein C₁- bis C₄-Monohydroxyalkylradikal, ein C₂- bis C₄-Polyhydroxyalkylradikal, ein C₁- bis C₄-Aminoalkylradikal oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder ein C₁- bis C₄-Alkylradikal,
mit der Maßgabe, daß die Verbindungen der Formel (3) nur eine Verbrückung Y pro Molekül enthalten.

Die in Formel (3) verwendeten Substituenten sind erfindungsgemäß analog zu den obigen Ausführungen definiert.

Bevorzugte zweikernige Entwicklerkomponenten der Formel (3) sind insbesondere: N,N'-bis-(β-Hydroxyethyl)-N,N'-bis-(4'-Aminophenyl)-1,3-diamino-propanol, N,N'-bis-(β-Hydroxyethyl)-N,N'-bis-(4'-aminophenyl)-ethylendiamin, N,N'-bis-(4-Aminophenyl)-tetramethylendiamin, N,N'-bis-(β-Hydroxyethyl)-N,N'-bis-(4-aminophenyl)-tetramethylendiamin, N,N'-bis-(4-Methylaminophenyl)-tetramethylendiamin, N,N'-bis-(Ethyl)-N,N'-bis(4'-amino,3'-methylphenyl)-ethylendiamin, 1,8-bis-(2,5-Diaminophenoxy)-3,5-dioxaoktan, Bis-(2-hydroxy-5-aminophenyl)-methan, 1,4-Bis-(4-aminophenyl)-diaza-cycloheptan und 1,10-Bis-(2,5-diaminophenyl)-1,4,7,10-tetraoxadecan und ihre physiologisch verträglichen Salze.

Ganz besonders bevorzugte zweikernige Entwicklerkomponenten der Formel (3) sind N,N'-bis-(β-Hydroxyethyl)-N,N'-bis-(4'-aminophenyl)-1,3-(diamino-propanol, Bis-(2-hydroxy-5-aminophenyl)-methan, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (4) 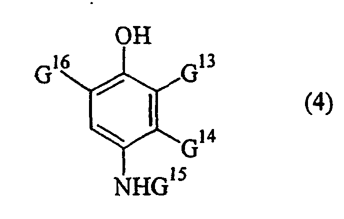 wobei:
- G¹³ steht für ein Wasserstoffatom, ein Halogenatom, ein C₁- bis C₄-Alkylradikal, ein C₁- bis C₄-Monohydroxyalkylradikal, ein (C₁- bis C₄)-Alkoxy-(C₁- bis C₄)-alkylradikal, ein C₁- bis C₄-Aminoalkylradikal, ein Hydroxy-(C₁- bis C₄)-alkylaminoradikal, ein C₁- bis C₄-Hydroxyalkoxyradikal, ein C₁- bis C₄-Hydroxyalkyl-(C₁-bis C₄)-aminoalkylradikal oder ein (Di-C₁- bis C₄-Alkylamino)-(C₁- bis C₄)-alkylradikal, und
- G¹⁴ steht für ein Wasserstoff- oder Halogenatom, ein C₁- bis C₄-Alkylradikal, ein C₁- bis C₄-Monohydroxyalkylradikal, ein C₂- bis C₄-Polyhydroxyalkylradikal, ein (C₁- bis C₄)-Alkoxy-(C₁- bis C₄)-alkylradikal, ein C₁- bis C₄-Aminoalkylradikal oder ein C₁- bis C₄-Cyanoalkylradikal,
- G¹⁵ steht für Wasserstoff, ein C₁- bis C₄-Alkylradikal, ein C₁- bis C₄-Monohydroxyalkylradikal, ein C₂- bis C₄-Polyhydroxyalkylradikal, ein Phenylradikal oder ein Benzylradikal, und
- G¹⁶ steht für Wasserstoff oder ein Halogenatom.

Die in Formel (4) verwendeten Substituenten sind erfindungsgemäß analog zu den obigen Ausführungen definiert.

Bevorzugte p-Aminophenole der Formel (4) sind insbesondere p-Aminophenol, N-Methyl-p-Aminophenol, 4-Amino-3-methyl-phenol, 4-Amino-3-fluorphenol, 2-Hydroxymethylamino-4-amino-phenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-fluorophenol, 4-Amino-2-chlorphenol, 2,6-Dichlor-4-aminophenol, 4-Amino-2-((diethylamino)methyl)phenol sowie ihre physiologisch verträglichen Salze.

Ganz besonders bevorzugte Verbindungen der Formel (4) sind p-Aminophenol, 4-Amino-3-methylphenol, 4-Amino-2-aminomethylphenol und 4-Amino-2-((diethylamino)methyl)phenol.

Ferner kann die Entwicklerkomponente ausgewählt sein aus o-Aminophenol und seinen Derivaten, wie beispielsweise 2-Amino-4-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorobenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl)amino-1-methylpyrazol.

Bevorzugte pyrazol-Pyrimidin-Derivate sind insbesondere die Derivate des Pyrazol-[1,5-a]-pyrimidin der folgenden Formel (5) und dessen tautomeren Formen, sofern ein tautomerisches Gleichgewicht besteht: 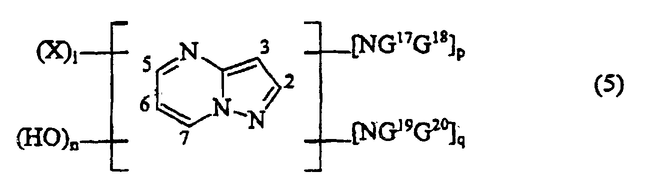 wobei:
- G¹⁷, G¹⁸, G¹⁹ und G²⁰ unabhängig voneinander stehen für ein Wasserstoffatom, ein C₁- bis C₄-Alkylradikal, ein Aryl-Radikal, ein C₁- bis C₄-Hydroxyalkylradikal, ein C₂- bis C₄-Polyhydroxyalkylradikal ein (C₁- bis C₄)-Alkoxy-(C₁- bis C₄)-alkylradikal, ein C₁- bis C₄-Aminoalkylradikal, das gegebenenfalls durch ein Acetyl-Ureid- oder Sulfonyl-Radikal geschützt sein kann, ein (C₁- bis C₄)-Alkylamino-(C₁- bis C₄)-alkylradikal, ein Di-[(C₁- bis C₄)-alkyl]-(C₁- bis C₄)-aminoalkylradikal, wobei die Dialkyl-Radikale gegebenenfalls einen Kohlenstoffzyklus oder einen Heterozyklus mit 5 oder 6 Kettengliedern bilden, ein C₁- bis C₄-Hydroxyalkyl- oder ein Di-(C₁- bis C₄)-[Hydroxyalkyl]-(C₁- bis C₄)-aminoalkylradikal,
- die X-Radikale stehen unabhängig voneinander für ein Wasserstoffatom, ein C₁- bis C₄-Alkylradikal, ein Aryl-Radikal, ein C₁- bis C₄-Hydroxyalkyladikal, ein C₂- bis C₄-Polyhydroxyalkylradikal, ein C₁- bis C₄-Aminoalkylradikal, ein (C₁- bis C₄)-Alkylamino-(C₁- bis C₄)-alkylradikal, ein Di-[( C₁- bis C₄)alkyl]- (C₁- bis C₄)-aminoalkylradikal, wobei die Dialkyl-Radikale gegebenenfalls einen Kohlenstoffzyklus oder einen Heterozyklus mit 5 oder 6 Kettengliedern bilden, ein C₁- bis C₄-Hydroxyalkyl- oder ein Di-(C₁- bis C₄-hydroxyalkyl)-aminoalkylradikal, ein Aminoradikal, ein C₁- bis C₄-Alkyl- oder Di-(C₁- bis C₄-hydroxyalkyl)-aminoradikal, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (5) verwendeten Substituenten sind erfindungsgemäß analog zu den obigen Ausführungen definiert.

Wenn das Pyrazol-[1,5-a]-pyrimidin der obenstehenden Formel (5) eine Hydroxygruppe an einer der Positionen 2, 5 oder 7 des Ringsystems enthält, besteht ein tautomeres Gleichgewicht, das zum Beispiel im folgenden Schema dargestellt wird:

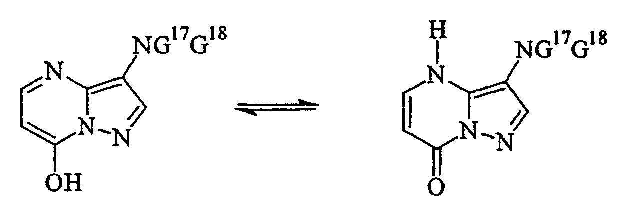

Unter den Pyrazol-[1,5-a]-pyrimidinen der obenstehenden Formel (5) kann man insbesondere nennen:
- Pyrazol-[1,5-a]-pyrimidin-3,7-diamin;
- 2,5-Dimethyl pyrazol-[1,5-a]-pyrimidin-3,7-diamin;
- Pyrazol-(1,5-a]-Pyrimidin-3,5-diamin;
- 2,7-Dimethyl pyrazol-[1,5-a]-pyrimidin-3,5-diamin;
- 3-Amino pyrazol-[1,5-a]-pyrimidin-7-ol;
- 3-Amino pyrazol-[1,5-a]-pyrimidin-5-ol;
- 2-(3-Amino pyrazol-[1,5-a]-pyrimidin-7-ylamino)-ethanol;
- 2-(7-Amino pyrazol-[1,5-a]-pyrimidin-3-ylamino)-ethanol;
- 2-[(3-Amino pyrazol-[1,5-a]-pyrimidin-7-yl)-(2-hydroxy-ethyl)-amino]-ethanol;
- 2-[(7-Amino pyrazol-[1,5-a]-pyrimidin-3-yl)-(2-hydroxy-ethyl)-amino]-ethanol;
- 5,6-Dimethyl pyrazol-[1,5-a]-pyrimidin-3,7-diamin;
- 2,6-Dimethyl pyrazol-[1,5-a]-pyrimidin-3,7-diamin;
- 2,5, N7, N7-Tetramethyl pyrazol-[1,5-a]-pyrimidin-3,7-diamin;
sowie ihre physiologisch verträglichen Salze und ihre tautomeren Formen, wenn ein tautomerisches Gleichgewicht vorhanden ist.

Die Pyrazol-[1,5-a]-pyrimidine der obenstehenden Formel (5) können wie in der Literatur beschrieben durch Zyklisierung ausgehend von einem Aminopyrazol oder von Hydrazin hergestellt werden.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind:
- m-Aminophenol und dessen Derivate wie beispielsweise 5-Amino-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol,

Besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die Oxidationsfarbstoffvorprodukte sind in den erfindungsgemäßen Mitteln bevorzugt in Mengen von 0,01 bis 20 Gew.-%, vorzugsweise 0,5 bis 5 Gew.-%, jeweils bezogen auf das gesamte Mittel, enthalten.

Prinzipiell können die Oxidationsfarbstoffvorprodukte gemeinsam mit den Verbindungen der Formel (1) formuliert werden. Es kann aber auch erfindungsgemäß bevorzugt sein, die Verbindungen der Formel (1) getrennt von den Oxidationsfarbstoffen, insbesondere den aminogruppenhaltigen Aromaten und Heterozyklen, zu lagern, so daß eine vorzeitige Reaktion dieser Komponenten mit den Verbindungen der Formel (1) ausgeschlossen werden kann. So können diese Verbindurigen in zwei getrennten wäßrigen Medien, wie beispielsweise Emulsionen gelagert werden, die erst unmittelbar vor der Anwendung auf den Fasern vereinigt werden. Ferner kann auch eine Komponente in einem wäßrigen Medium und die andere Komponente als Pulver konfektioniert sein, das unmittelbar vor der Anwendung zu dem wäßrigen Medium gegeben wird. Im Rahmen dieser Konfektionierung kann es erfindungsgemäß bevorzugt sein, die Komponente enthaltend die Verbindungen der Formel (1) als Pulver zu formulieren. Ferner kann eine vorzeitige Reaktion auch vermieden werden, wenn die beiden Komponenten gemeinsam als Pulverhaarfarbe formuliert werden. Die Komponenten werden dann erst unmittelbar vor der Anwendung auf den Fasern durch Zugabe einer wäßrigen Komponente aktiviert. Abschließend sei auch noch die Möglichkeit erwähnt, die beiden Komponenten getrennt in einem inerten Medium, wie beispielsweise einem inerten Öl, zu dispergieren.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (6a), 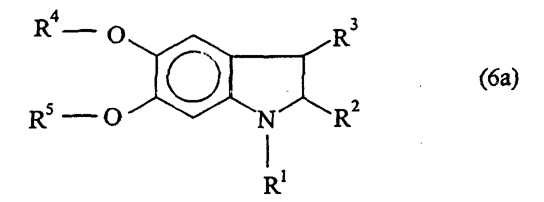 in der unabhängig voneinander
R¹ steht für Wasserstoff, eine C₁-C₄-Alkylgruppe oder eine C₁-C₄-Hydroxy-alkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (6b), 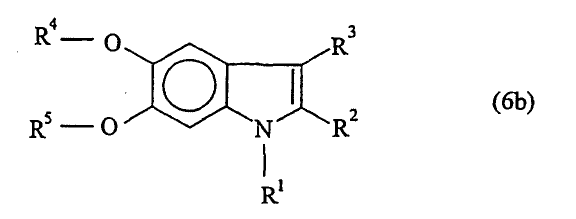 in der unabhängig voneinander
R¹ steht für Wasserstoff, eine C₁-C₄-Alkylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

Insbesondere bei der Verwendung von Farbstoff-Vorstufen vom Indolin- oder Indol-Typ hat es sich als vorteilhaft erwiesen, als Alkalisierungsmittel eine Aminosäure und/oder ein Oligopeptid einzusetzen.

In einer weiteren Ausführungsform der vorliegenden Erfindung können die erfindungsgemäßen Mittel, enthaltend die Verbindungen der Formel (I) sowie weitere Farbstoffvorprodukte, auch im Rahmen eines zweistufigen Verfahrens angewendet werden. So ist eine Vorprenetration mit den Verbindungen der Formeln (I) oder den Farbstoff-vorprodukten denkbar. Die Vorprenetation kann gegebenenfalls in der Gegenwart eines Reduktionsmittels erfolgen. Weiterhin kann gegebenenfalls nach Auftragung der jeweils zweiten Komponente ein zusätzliche Oxidation erfolgen.

Neben den Farbstoffvorprodukten können die erfindungsgemäßen Mittel zur weiteren Nuancierung direktziehende Farbstoffe enthalten. Diese sind üblicherweise ausgewählt aus Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 26, Basic Blue 99, HC Violet 1, Disperse Violet 1, Disperse Violet 4, Basic Violet 2, Basic Violet 14, Acid Violet 43, Disperse Black 9, Acid Black 52, Basic Brown 16 und Basic Brown 17 bekannten Verbindungen sowie 1,4-Bis-(β-hydroxyethyl)-amino-2-nitrobenzol, 3-Nitro-4-(β-hydroxyethyl)-aminophenol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Die Vielfalt der erzielbaren Farbnuancen des erfindungsgemäßen Färbesystems kann durch Kombination mit einer oder mehreren Verbindungen mit primärer oder sekundärer Amino- oder Hydroxygruppe, ausgewählt aus der Gruppe der Aminosäuren und Peptide, der aromatischen Amine, Phenole, Aminophenole sowie stickstoffhaltigen Heterocyclen weiter erhöht werden. Dabei werden in vielen Fällen auch dunklere Nuancen erhalten.

Geeignete Aminosäuren sind insbesondere die natürlich vorkommenden und synthetischen Aminosäuren, beispielsweise Arginin, Histidin, Phenylalanin, Dihydroxyphenylalanin, Ornithin, Lysin. Geeignete Peptide sind vor allem Oligo- und Polypeptide, die eine ausreichende Wasserlöslichkeit in den erfindungsgemäßen Zubereitungen zur Keratinreduktion aufweisen. Als Beispiele sind Glutathion oder die in den Hydrolysaten von Kollagen, Keratin, Elastin, Casein oder Pflanzenproteinen, wie Sojaprotein, Weizenprotein, Algenprotein oder Mandelprotein, enthaltenen Oligopeptide zu nennen.

Geeignete aromatische Amine und Aminophenole sind N,N-Dimethyl-, N,N-Diethyl-, N-(2'-Hydroxyethyl)-N-ethyl-, N,N-Bis-(2'-hydroxyethyl)-, N-(2'-Methoxyethyl)-, 2-Chlor-, 2,3-, 2,4-und 2,5-Dichlor-p-phenylendiamin, 2,5-Dihydroxy-4-morpholinoanilin-dihydro-bromid, 2-, 3- und 4-Aminophenol, 2-Aminomethyl-4-aminophenol, 2-Hydroxymethyl-4-aminophenol, o- und p-Phenylendiamin, o- und m-Toluylendiamin, 2,5-Diamino-phenol, -toluol und -phenethol, 4-Amino-3-methylphenol, 2-(2',5'-Diaminophenyl)-ethanol, 2,4-Diaminophenoxy-ethanol, 2-(2',5'-Diaminophenoxy)-ethanol, 4-Methylamino-, 3-Amino-4-(2'hydroxyethyloxy)-, 3,4-Methylendiamino- und 3,4-Methylendioxyanilin, 3-Amino-2,4-dichlor-, 4-Methylamino-, 2-Methyl-5-amino-, 3-Methyl-4-amino-, 2-Methyl-5-(2'-hydro-xyethylamino)-, 6-Methyl-3-amino-2-chlor-, 2-Methyl-5-amino-4-chlor-, 3,4-Methylen-dioxy-, 5-(2'-Hydroxyethylamino)-4-methoxy-2-methyl- und 4-Amino-2-hydroxymethyl-phenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfansäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, aromatische Aniline beziehungsweise Phenole mit einem weiteren aromatischen Rest, wie sie in der Formel (7) dargestellt sind 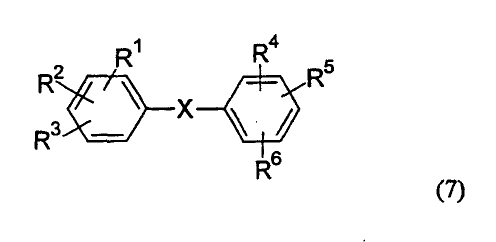 in der R¹ für eine Hydroxy- oder eine Aminogruppe, die durch C₁₋₄-Alkyl, C₁₋₄-Hydroxyalkyl- oder C₁₋₄-Alkoxy-C₁₋₄-alkyl substituiert sein kann, steht,
R², R³, R⁴, R⁵ und R⁶ für Wasserstoff, eine Hydroxy- oder eine Aminogruppe, die durch C₁₋₄-Alkyl-, C₁₋₄-Hydroxyalkyl, C₁₋₄-Aminoalkyl- oder C₁₋₄-Alkoxy-C₁₋₄-alkylgruppe substituiert sein kann für eine Carbon- oder Sulfonsäuregruppe stehen, und
X für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel (8)

Z-(CH₂-Y-CH₂-Z')ₒ (8),

in der Y eine direkte Bindung, eine CH₂- oder CHOH-Gruppe bedeutet,
Z und Z' unabhängig voneinander ein Sauerstoffatom, eine NR⁷-Gruppe, worin R⁷ Wasserstoff, eine C₁₋₄-Alkyl- oder Hydroxy-C₁₋₄-alkylgruppe bedeutet, die Gruppe -O-(CH₂)ₚ-NH oder NH-(CH₂)_{p'}-O, worin p und p' 2 oder 3 sind, stehen und
o eine Zahl von 1 bis 4 bedeutet,
wie beispielsweise 4,4'-Diaminostilben, 4,4'-Diaminostilben-2,2'-disulfonsäure-mono- oder -di-Na-Salz, 4,4'-Diaminodiphenylmethan, -sulfid, -sulfoxid, -amin, 4,4'-Diaminodiphenyl-amin-2-sulfonsäure, 4,4'-Diaminobenzophenon,. -diphenylether, 3,3',4,4'-Tetraaminodi-phenyl, 3,3',4,4'-Tetraamino-benzophenon, 1,3-Bis-(2',4'-diaminophenoxy)-propan, 1,8-Bis-(2',5'-diaminophenoxy)-3,6-dioxaoctan, 1,3-Bis-(4'-aminophenylamino)-propan, -2-propa-nol, 1,3-Bis-[N-(4'aminophenyl)-2-hydroxyethylamino]-2-propanol, N,N-Bis-[2-(4'-amino-phenoxy)-ethyl]-methylamin, N-Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete Phenole sind beispielsweise das 2-, 3- oder 4-Methoxy-, das 3-Dimethylamino-, 2-(2'-Hydroxyethyl)- und das 3,4-Methylendioxy-phenol, das Resorcin und das 2-, 4- und 5-Methylresorcin, das 2- und 4-Chlorresorcin, 2,5-Dimethylresorcin, Brenzkatechin, Hydrochinon, Pyrogallol, Phloroglucin, Hydroxyhydrochinon, die 2,4- oder 3,4-Dihy-droxybenzoe- oder - phenylessigsäure, die Gallussäure, die 2,4,6-Trihydroxybenzoesäure oder das 2,4,5-Trihydroxyacetophenon, das 1-Naphthol, das 1,5-, 2,3- und 2,7-Dihydroxynaphthalin, die 6-Dimethylamino-4-hydroxy-2-naphthalinsulfonsäure oder die 3,6-Dihy-droxy-2,7-naphthalindisulfonsäure.

Geeignete stickstoffhaltige heterocyclische Verbindungen sind beispielsweise 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino- und 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino- und 2-Amino-4-methoxy-6-methyl-pyrimidin, 3-Amino-, 3-Amino-5-hydroxy- und 3,5-Diaminopyrazol, 2-,3-, 8-Aminochinolin, 4-Aminochinaldin, 2-, 6-Aminonicotinsäure, 5-Aminoisochinolin, 5-, 6-Aminoindazol, 5- und 7-Aminobenzimidazol und -benzothiazol, 2,5-Dihydroxy-4-morpholinoanilin sowie Indol- und Indolinderivate, wie 4-, 5-, 6- und 7-Aminoindol, 5,6-Dihydroxyindol, 5,6-Dihydroxyindolin und 4-Hydroxyindolin. Die vorgenannten Verbindungen können sowohl in freier Form als auch in Form ihrer physiologisch verträglichen Salze, beispielsweise als Salze anorganischer Säuren, wie Salz- oder Schwefelsäure, eingesetzt werden.

Diese Färbesysteme können noch weiter verstärkt werden durch geeignete stickstoffhaltige Heterocyclen wie beispielsweise Piperidin, Piperidin-2-, -3- oder -4-carbonsäure, Pyridin, Pyridin-2-carbonsäure, Pyridin-3-carbonsäure, Pyridin-4-carbonsäure, 2-, 3- oder 4-Hydroxypyridin, Imidazol, 1-Methylimidazol, Histidin, Pyrrolidin, Pyrrolidon, Pyrrolidon-5-carbonsäure, Pyrazol, 1,2,4-Triazol, Piperazin sowie deren physiologisch verträglichen Salze.

Die erfindungsgemäßen Mittel enthalten die Verbindungen der Formel (1) bevorzugt in einem geeigneten wäßrigen, alkoholischen oder wäßrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Verbindungen der Formel (1) in eine pulverförmige oder auch Tabletten-förmige Formulierung zu integrieren.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzufiihren, wobei die Enzyme sowohl zur Erzeugung von oxidierenden Per-Verbindungen eingesetzt werden als auch zur Verstärkung der Wirkung einer geringen Menge vorhandener Oxidationsmittel. So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase, Ascorbat-Oxidase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die den erfindungsgemäßen Mittel können weiterhin alle für solche Zubereitungen bekannten Wirk-, Zusatz- und Hilfsstoffe enthalten. In vielen Fällen enthalten diese Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quatemium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die im Rahmen des erfindungsgemäßen Verfahrens eingesetzten Mittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat®100 (Poly(dimethyldiallylammoniutnchlorid)), Merquat®550 (Dimethyldiallylammoniumchlorid-Acrylamid-Copolymer) und Merquat® 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quatemierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Coming; ein stabilisiertes Trimethylsilylamodimethicon), Dow Corning® 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkemöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und saure Aminosäuren sowie Basen und alkalische Aminosäuren,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrytamid-Copolymere
- Perlglanzmittel wie Ethyleriglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein zweiter Gegenstand der vorliegenden Erfindung ist ein Verfahren zum Färben keratinischer Fasern, bei dem ein Mittel, enthaltend in einem kosmetisch akzeptablen Medium als Farbstoffvorprodukt mindestens ein spezielles Derivat des Benzo[b]furan-3-ons und/oder mindestens ein spezielles Derivat des Benzo[b]thiophen-3-ons und/oder deren Keto-Enol-Tautomeren, auf die Fasern aufgetragen wird und nach einer Einwirkungszeit wieder abgespült wird.

Ein dritter Gegenstand der vorliegenden Erfindung ist die Verwendung von speziellen Derivaten des Benzo[b]furan-3-ons, des Benzo[b]thiophen-3-ons und/oder deren Keto-Enol-Tautomeren zur Färbung keratinischer Fasern.

Weiterhin sind die Verbindungen 5-Carboxymethoxy-benzo[b]furan-3-on, 5-(2'-Hydroxyethoxy)-benzo[b]furan-3-on, 5-Carboxymethoxy-benzo[b]thiophen-3-on, 5,6-Dihydroxy-benzo[b]thiophen-3-on, 6-Hydroxy-5-methoxy-benzo[b]thiophen-3-on neu und somit Gegenstand der vorliegenden Anmeldung.

Die folgenden Beispiele sollen den Gegenstand der vorliegenden Anmeldung verdeutlichen.

### Ausführungsbeispiele

### 1 Synthese der 3-Hydroxy-5-oxy-benzo[b]thiophene (Tautomere der 5-Oxy-benzo[b]thiophen-3-one)

### 1.1 Synthese von 3,5-Dihydroxy-benzo[b]thiophen (I)

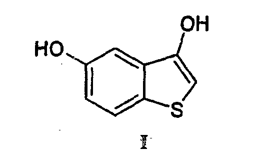

Diese Verbindung wurde gemäß A. Pawlik, *Dissertation,* Techn. Universität Braunschweig **1995,** 98-101 hergestellt.

### 1.2 Synthese des 5-Carboxymethoxy-3-hydroxy-benzo[b]thiophens

Dieses Molekül wurde nach Syntheseschema 1 hergestellt:

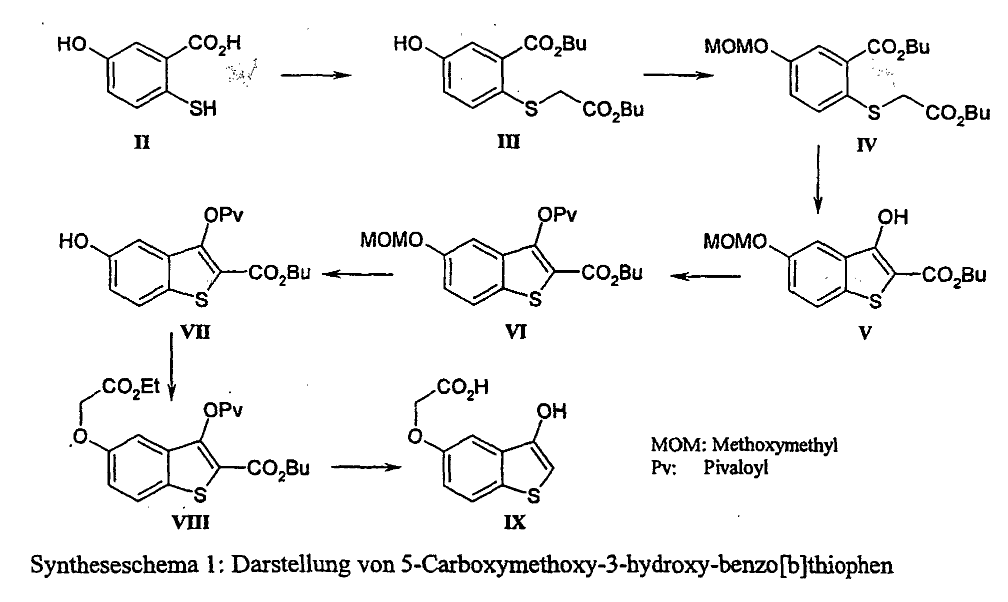

### 1.2.1 Synthese von 5-Hydroxy-2-mercapto-benzoesäure (II)

Diese Verbindung wurde gemäß A. Pawlik, *Dissertation,* Techn. Universität Braunschweig **1995,** 98-101 hergestellt.

### 1.2.2 Synthese von 5-Hydroxy-2-butoxycarbonylmethylsulfanyl-benzoesäurebutylester (III)

83.40 g (490 mmol) **II**, 196.00 g (4.90 mol) NaOH und 102.13 g (735 mmol) Bromessigsäure wurden in 1.5 L MeOH und 600 mL Wasser 32 h unter Rückfluß erhitzt. Anschließend wurde mit konzentrierter HCl pH 1 eingestellt, bevor die Lösemittel im Vakuum entfernt wurden. Der Rückstand wurde mit 1.5 L *n*-Butanol und 15 mL konzentrierter H₂SO₄ 12 h am Wasserabscheider erhitzt. Nach Entfernen des Lösemittels wurde der Rückstand mit 1 L Chloroform aufgenommen, einmal mit Wasser gewaschen und über Na₂SO₄ getrocknet. Das Lösemittel wurde entfernt. Nach Lösen des Rückstandes in Ethylacetat erhielt man durch Ausfällen mit *n*-Pentan 101.80 g (299 mmol, 61 %) **III**.

### 1.2.3 Synthese von 5-Methoxymethyloxy- 2-butoxycarbonylmethylsulfanylbenzoesäurebutylester (IV)

Unter einer mit 3 A Molsieb gefüllten Extraktionshülse wurden 1.70 g (5 mmol) **III**, 2.21 mL (25 mmol) Dimethoxymethan und eine Spatelspitze *p*-Toluolsulfonsäure in 75 mL Dichlormethan 72 h unter Rückfluß erhitzt. Anschließende Säulenfiltration (50 g SiO_{2,} *n*-Hexan : Ethylacetat =5:1) lieferte 1.61 g (4.2 mmol, 84 %) **IV**.

### 1.2.4 Synthese von 2-Butoxycarbonyl-3-hydroxy-5-methoxymethyloxy-bezo[b]thiophen (V)

In 80 mL DMF wurden 6.20 g (16 mmol) **IV** und 0.80 g (20 mmol, 60 %) NaH 5 h bei Raumtemperatur gerührt. Das Lösemittel wurde entfernt und der Rückstand mit 250 mL CHCl₃ aufgenommen, dreimal mit Wasser gewaschen und über Na₂SO₄ getrocknet. Nach Umkristallisation aus *n*-Hexan erhielt man 4.22 g (13.6 mmol, 85 %) **V.**

### 1.2.5 Synthese von 2-Butoxycarbonyl-5-methoxymethyloxy-3-pivaloyloxy-benzo[b]thiophen (VI)

6.21 g (20 mmol) **V** und 3.70 mL (30 mmol) Pivaloylchlorid wurden 12 h bei Raumtemperatur in 150 mL Pyridin gerührt. Nach Entfernen des Lösemittels lieferte eine Flash-Chromatographie (200 g SiO₂, *n*-Hexan : Ethylacetat = 5 : 1 + 1% Essigsäure, mit CHCl₃ aufgetragen) 7.39 g (18.7 mmol, 94 %) **VI**.

### 1.2.6 Synthese von 2-Butoxycarbonyl-5-hydroxy-3-pivaloyloxy-benzo[b]thiophen (VII)

In 150 mL Essigsäure wurden 5.92 g (15 mmol) VI mit einigen Tropfen konzentrierter HCl versetzt und 10 h bei Raumtemperatur gerührt. Anschließend wurden zur Reaktionslösung 500 mL Wasser gegeben und mehrfach mit Et₂O extrahiert. Die vereinigten organischen Phasen wurden über Na₂SO₄ getrocknet und vom Lösemittel befreit. Eine Umkristallisation aus Diethylether : *n*-Hexan = 4 : 1 lieferte 4.21 g (12 mmol, 80 %) **VII**.

### 1.2.7 Synthese von 2-Butoxycarbonyl-5-ethoxycarbonylmethoxy-3-pivaloyloxybenzo[b]thiophen (VIII)

3.50 g (10 mmol) VII, 2.22 mL (20 mmol) Bromessigsäureethylester und 2.48 g (25 mmol) K₂CO₃ wurden in 250 mL Aceton 5 h unter Rückfluß erhitzt. Die Reaktionsmischung wurde filtriert. Das Filtrat wurde vom Lösemittel befreit. Nach Umkristallisation aus n-Hexan erhielt man 4.00 g (9.2 mmol, 92 %) **VIII**.

### 1.2.8 Synthese von 5-Carboxymethoxy-3-hydroxy-benzo[b]thiophens (IX)

In 300 mL entgastem Wasser wurden 1.96 g (4.5 mmol) **VIII** und 2.70 g (67.5 mmol) NaOH 15 h unter Rückfluß erhitzt. Anschließend wurde mit konzentrierter HCl pH 1 eingestellt und die wäßrige Phase dreimal mit Ethylacetat extrahiert. Die vereinigten organischen Phasen werden über Na₂SO₄ getrocknet. Eine Flash-Chromatographie (200 g SiO₂, Ethylacetat : Essigsäure = 100 : 1, Präadsorption: 10 g SiO₂/Ethanol) liefert 801 mg (3.6 mmol, 79 %) **IX**.

### 2 Synthese der 5,6-Dioxy -3-hydroxy- benzo[b]thiophen-Derivate (Tautomere der 5, 6-Dioxybenzo[b]thiophen-3-on-Derivate)

Die 5,6-Dioxy-3-hydroxy-benzo[b]thiophen-Denvate wurden gemäß Syntheseschema 2 hergestellt.

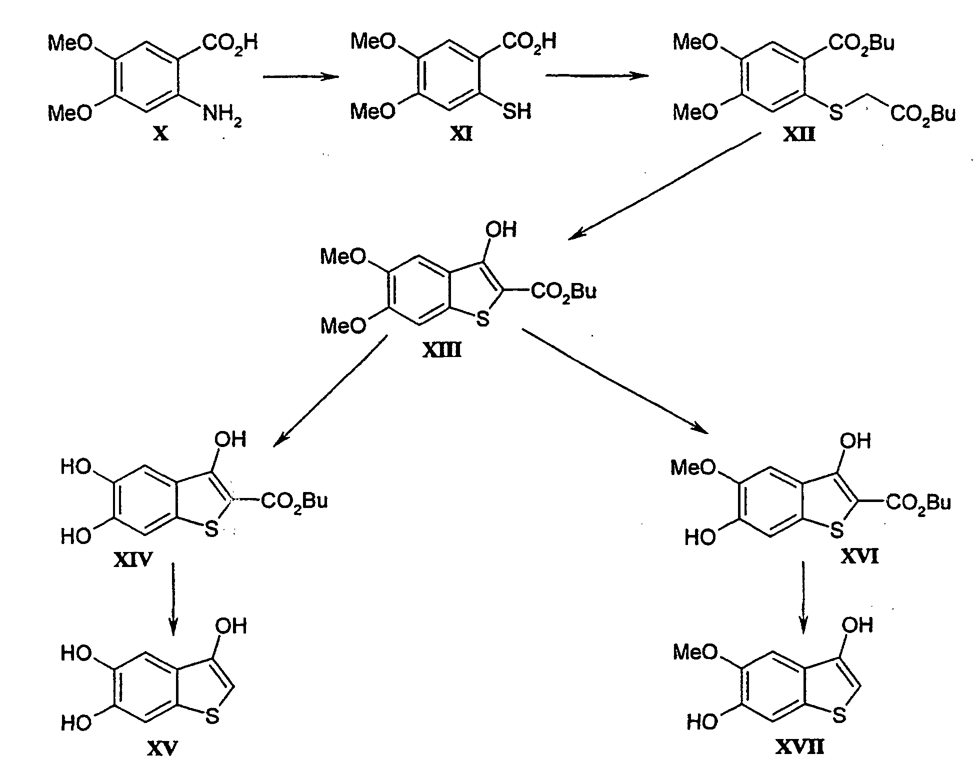

### 2.1 Synthese von 3,5,6-Trihydroxy-benzo[b]thiophen (XV)

### 2.1.1 Synthese von 4,5-Dimethoxy-2-mercapto-benzoesäure (XI)

Diese Verbindung wurde in Anlehnung an J. Szabò et al., *Acta Chim. Acad. Sci. Hung*. **1958,** *17*, 201-209 hergestellt.

### 2.1.2 Synthese von 4, 5-Dimethoxy-2-butoxycarbonylmethylsulfanyl -benzoesäurebutylester (XII)

In einer Lösung aus 50 mL MeOH und 25 mL Wasser wurden 2.36 g (11 mmol) **XI**, 2.29 g (17 mmol) Bromessigsäure und 4.40 g (110 mmol) NaOH 24 h unter Rückfluß gerührt.

Nach Ansäuern mit konzentrierter HCl auf pH 1 wurden die Lösemittel im Vakuum entfernt und der Rückstand mit 50 mL n-Butanol und 2 mL konzentrierter H₂SO₄ weitere 60 h am Wasserabscheider erhitzt.

Die Reaktionsmischung wurde vom Lösemittel befreit und der Rückstand mit 100 mL CHCl₃ aufgenommen. Nach Waschen der organischen Phase mit Wasser und Trocknen über Na₂SO₄ lieferte eine Flash-Chromatographie (100 g SiO₂, *n*-Hexan : Ethylacetat = 10 : 1 + 1 % Essigsäure) 3.80 g (9.9 mmol, 90 %) **XII.**

### 2.1.3 Synthese von 2-Butoxycarbonyl-5,6-dimethoxy-3-hydroxy-benzo[b]thiophen (XIII)

19.22 g (50 mmol) **XII** wurden mit 2.75 g (63 mmol, 60 %) NaH in 250 mL DMF 3.5 h bei Raumtemperatur gerührt. Anschließend hydrolysierte man mit 50 mL gesättigter NH₄Cl-Lösung und entfernte die Lösemittel. Der Rückstand wurde in 500 mL CHCl₃ aufgenommen, filtriert, mit Wasser gewaschen und über Na₂SO₄ getrocknet. Nach Flash-Chromatographie (250 g SiO₂, *n*-Hexan : Ethylacetat = 5 : 1 + 1 % Essigsäure) erhielt man 14.70 g (47 mmol, 95 %) **XIII**.

### 2.1.4 Synthese von 2-Butoxycarbonyl-3,5,6-trihydroxy-benzo[b]thiophen (XIV)

Bei 0 °C wurden 2.79 g (9.0 mmol) **XIII** in 150 mL CH₂Cl₂ mit 2.64 mL (27 mmol) BBr₃ 2 h gerührt. Nach Hydrolyse mit 75 mL Wasser wurde mit Ethylacetat extrahiert. Die vereinigten organischen Phasen wurden über Na₂SO₄ getrocknet und man erhielt nach Flash-Chromatographie (200 g SiO₂, *n*-Hexan : Ethylacetat = 2 : 1 + 1 % Essigsäure) 2.48 g (8.8 mmol, 98 %) **XIV**.

### 2.1.5 Synthese von 3,5,6-Trihydroxy-benzo[b]thiophen (XV)

In 300 mL entgastem Wasser wurden 2.82 g (10 mmol) **XIV** und 6.00 g (150 mmol) NaOH 3.5 h unter Rückfluß erhitzt. Anschließend säuerte man die noch heiße Lösung mit konzentrierter HCl auf pH 1 an. Das beim Abkühlen im Eisbad ausfallende **XV** wurde abgetrennt und mit wenig Diethylether gewaschen. Man erhielt 1.57 g (8.6 mmol, 86 %) **XV**.

### 2.2 Synthese von 5-Methoxy-3,6-dihydroxy-benzo[b]thiophen (XVII)

### 2.2.1 Synthese von 2-Butoxycarbonyl-3,6-dihydroxy-5-methoxy-benzo[b]thiophen (XVI)

4.45 mL (60 mmol) Ethanthiol wurden in 100 mL DMF mit 2.62 g (60 mmol, 60 %) NaH 15 min bei Raumtemperatur gerührt. Zu dieser Lösung wurden 9.31 g (30 mmol) **XIII** gegeben und 4 h unter Rückfluß erhitzt. Nach Hydrolyse mit 25 mL gesättigter NH₄Cl-Lsg wurden die Lösemittel im Vakuum entfernt, der Rückstand mit 500 mL Ethylacetat aufgenommen und mit Wasser gewaschen. Nach Trocknen über Na₂SO₄ lieferte eine Flash-Chromatographie (250 g SiO₂, *n*-Hexan : Ethylacetat = 2 : 1 + 1 % Essigsäure, Präadsorption: 25 g SiO₂/CHCl₃) 6.89 g (23 mmol, 78 %) **XVI**.

### 2.2.2 Synthese von 3,6-Dihydroxy-5-methoxy-benzo[b]thiophen (XVII)

In 100 mL entgastem Wasser wurden 2.96 g (10 mmol) **XVI** und 6.00 g NaOH 3.5 h unter Rückfluß erhitzt. Anschließend wurde die noch heiße Lösung mit konzentrierter HCl auf pH 1 angesäuert. Das beim Abkühlen im Eisbad ausfallende **XVII** wurde abgetrennt und mit wenig *n*-Hexan gewaschen. Man erhielt 1.23 g (6.3 mmol, 63 % mmol) **XVII**.
Durch Extraktion des wäßrigen Filtrats mit Ethylacetat und anschließender Flash-Chromatographie (50 g SiO₂, *n*-Hexan : Ethylacetat = 2 : 1 + 1 % Essigsäure, Präadsorption: 2 g SiO₂/Ethylacetat) ließ sich die Ausbeute an **XVII** auf insgesamt 1.73 g (8.8 mmol, 88 %) steigern.

### 3 Synthese der 5-Oxy-benzo[b]furan-3-on-Derivate

Die 5-Oxy-benzo[*b*]furan-3-on-Derivate wurden gemäß Syntheseschema 3 hergestellt.

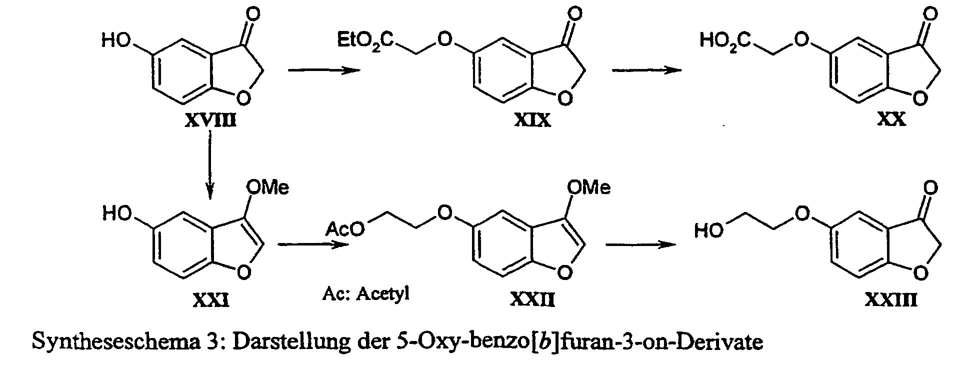

### 3.1 Synthese von 5-Hydroxy-benzo[b]furan-3-on (XVIII)

Das 5-Hydroay-benzo[*b*]furan-3-on (**XVIII**) wurde gemäß einer Vorschrift von M. C. Kloetzel et al., *J. Org. Chem.* **1955,** *20*, 38-49 synthetisiert.

### 3.2 Synthese von 5-Carboxymethoxy-benzo[b]furan-3-on (XX)

### 3.2.1 Synthese von 5-Ethoxycarbonylmethoxy-benzo[b]furan-3-on (XIX)

In 150 mL Tetrahydrofuran wurden 3.00 g (20 mmol) **XVIII**, 3.96 g (40 mmol) K₂CO₃ und 5.54 mL (50 mmol) Bromessigsäureethylester 72 h unter Rückfluß erhitzt. Anschließend wurde filtriert und das Lösemittel entfernt. Eine Flash-Chromatographie (200 g SiO₂, *n*-Hexan : Ethylacetat = 2 : 1 ) lieferte 4.63 g (19.6 mmol, 98 %) **XIX**.

### 3.2.2 Synthese von 5-Carboxymethoxy-benzo[b]furan-3-on (XX)

In einer Mischung aus 200 mL Tetrahydrofuran, 100 mL Wasser und 10 mL konzentrierter H₂SO₄ wurden 4.72 g (20 mmol) **XIX** 15 h unter Rückfluß erhitzt. Die Reaktionslösung wurde mit Ethylacetat und gesättigter NaCl-Lösung versetzt. Die Phasen wurden getrennt und die wäßrige dreimal mit Ethylacetat extrahiert. Die vereinigten organischen Phasen wurden über Na₂SO₄ getrocknet und vom Lösemittel befreit. Durch Flash-Chromatographie (200 g SiO₂, *n*-Hexan : Ethylacetat = 1 : 2 + 1 % Essigsäure, mit Ethanol aufgetragen) erhielt man 2.78 g (13 mmol, 67 %) **XX**.

### 3.3 Synthese von 5-(2'-Hydroxyethoxy-benzo[b]furan-3-on (XXIII)

### 3.3.1 Synthese von 5-Hydroxy-3-methoxy-benzo[b]furan (XXI)

900 mg (6.0 mmol) **XVIII**, 788 µl (7.2 mmol) Orthoameisensäuretrimethylester und 57 mg (0.3 mmol) *p*-Toluolsulfonsäure Monohydrat wurden in 70 mL Methanol 12 h unter Rückfluß erhitzt. Die Reaktion wurde durch Zugabe gesättigter NaHCO₃-Lösung gestoppt und die Lösemittel entfernt. Die anschließende Flash-Chromatographie (100 g SiO₂, *n*-Hexan : Ethylacetat = 2 : 1, mit Ethanol aufgetragen) lieferte 717 mg (4.4 mmol, 73 %) **XXI**.

### 3.3.2 Synthese von 3-Methoxy-5-(2'-Acetoxyethoxy)-benzo[b]furan (XXII)

Bei 0 °C wurden 164 mg (1.0 mmol) **XXI** und 48 mg (1.2 mmol, 60 %) NaH in 20 mL DMF 30 min gerührt. Zu dieser Suspension gab man 227 µl (2.0 mmol, 97 %) 2-Bromethylacetat und rührte 72 h bei Raumtemperatur. Durch Zugabe von 3 mL gesättigter NH₄Cl-Lösung quenchte man die Reaktion und entfernte die Lösemittel im Vakuum. Der Rückstand wurde mit Ethylacetat und Wasser aufgenommen, die wäßrige Phase noch zweimal mit Ethylacetat extrahiert und die vereinigten organischen Phasen über Na₂SO₄ getrocknet. Nach Säulenfiltration (40 g SiO₂, *n*-Hexan : Ethylacetat = 2 : 1, mit Ethylacetat aufgetragen) erhielt man 220 mg (0.88 mmol, 88 %) **XXII**.

### 3.3.3 Synthese von 5-(2'-Hydroxyethoxy)-benzo[b]furan-3-on (XXIII)

In einer Mischung aus 40 mL Tetrahydrofuran, 20 mL Wasser und 0.5 mL konz. H₂SO₄ wurden 751 mg (7.0 mmol) **XXII** 12 h unter Rückfluß gerührt. Die auf Raumtemperatur abgekühlte Reaktionsmischung wurde mit Ethylacetat und gesättigter NaCl-Lösung versetzt. Nach Trennen der Phasen wurde die wäßrige Phase noch zweimal mit Ethylacetat extrahiert und die vereinigten organischen Phasen über Na₂SO₄ getrocknet. Nach Säulenfiltration (50 g SiO₂, *n*-Hexan : Ethylacetat = 1 : 1 + 1 % Essigsäure, mit Ethylacetat aufgetragen) erhielt man 448 mg (2.3 mmol, 77 %) **XXIII**.

### 4 Anwendungsbeispiele

Die Mengenangaben verstehen sich, sofern nichts anderes angegeben ist, in Gewichtsprozent.

### 4.1 Versuche mit Verbindungen der Formel (1)

Zunächst wurden jeweils 0,1g Farbstoff mit 0,1g Natrosol® HR250 (Hydroxyethylcellulose, INCI-Bezeichnung: Hydroxyethylcellulose, Cognis) und 0,05g Ammoniumsulfat mit 9g Wasser versetzt und der pH-Wert mit einer 25%igen Ammoniaklösung auf einen Wert von 10 eingestellt. Anschließend wurde diese Quellung mit Wasser auf 10g aufgefüllt.

Unmittelbar vor der Anwendung wurden diese Lösungen für die Luftoxidation im Verhältnis 1:1 mit Wasser vermischt. Im Falle der H₂O₂-Oxidation wurden diese Lösungen entsprechend im Verhältnis 1:1 mit einer 3%igen wäßrigen Wasserstoffperoxidlösung (Marktprodukt Poly Color Tönungswäsche) vermischt.

In 10g dieser Anwendungszubereitung wurde je eine Haarsträhne (Alkinco Virgin-White, 2g) für 30min bei Raumtemperatur getaucht, anschließend mit Wasser gespült und getrocknet.

Die färberischen Ergebnisse sind in Tabelle I zusammengefaßt.

**Tabelle I**

| Verbindung | Farbnuance | |
|---|---|---|
| Nr. | Luftoxidation | H₂O₂-Oxidation |
| I | beige-olivbeige | hellbeige |
| IX | beigeblond | hellbeigeblond |
| XV | goldblond-beigeblond | hellgoldblond-hellbeigeblond |
| XVII | weizenblond | hellweizenblond |
| XVIII | goldblond | weizenblond |
| XX | Lichtgoldblond | lichtgoldblond |
| XXIII | Goldblond | hellgoldblond |

### 4.2 Versuche mit Verbindungen der Formel (1) und Oxidationsfarbstoffvorprodukten

Je 100mg 5-Hydroxy-benzo[b]furan-3-on (XVIII) und 100mg 2,4,5,6-Tetraaminopyrimidin-sulfat bzw. p-Toluylendiamin-sulfat sowie 65mg Natriummetabisulfit, 100mg Natrosol® HR 250 und 50mg Ammoniumsulfat wurden mit 9g Wasser versetzt und der pH-Wert mit einer 25%igen Ammoniaklösung auf einen Wert von 10 eingestellt. Anschließend wurde die Quellung mit Wasser auf 10g aufgefüllt. Je 2,5g dieser Quellungen wurden auf Haarsträhnen von ca. 0,5g Gewicht der Haarqualität Kerling naturweiß gegeben und in Aluminiumfolie eingewickelt. Die Farbentwicklung erfolgte sowohl bei Raumtemperatur als auch bei + 45°C. Nach 30 Minuten Einwirkzeit wurden die Strähnen gespült und getrocknet. Als Vergleich wurden analoge Versuche mit 2,4,5,6-Tetraaminopyrimidin-sulfat bzw. p-Toluylendiamin-sulfat ohne Zusatz von 5-Hydroxy-benzo[b]furan-3-on durchgeführt.

**Tabelle II:**

| **Rohstoff** | **% Rezeptur-Nr.** | | | |
|---|---|---|---|---|
| | **1 (Erfindung)** | **2 (Erfindung)** | **3 (Vergleich)** | **4 (Vergleich)** |
| 5-Hydroxybenzo[b]furan-3-on | 1,0 | 1,0 | - | - |
| p-Toluylendiaminsulfat | 1,0 | - | 1,0 | - |
| 2,4,5,6-Tetraaminopyrimidin-sulfat | - | 1,0 | - | 1,0 |
| Natriummetabisulfit | 0,65 | 0,65 | 0,65 | 0,65 |
| Ammoniumsulfat | 0,5 | 0,5 | 0,5 | 0,5 |
| Natrosol® 250 HR | 1,0 | 1,0 | 1,0 | 1,0 |
| Ammoniak, 25%ig | ad pH 10 | ad pH 10 | ad pH 10 | ad pH 10 |
| Wasser | ad 100 | ad 100 | ad 100 | ad 100 |
| Ausfärbung bei Raumtemperatur | Kupferfarben Farbtiefe mittelblond | Leuchtendes Goldblond Farbtiefe hellblond | Haselnuß Farbtiefe mittelblond | Leuchtendes Orangegelb Farbtiefe lichtblond |
| Ausfärbung bei 45°C | Rötliches Kupfer Farbtiefe mittelblond | Leuchtendes rötliches Goldblond Farbtiefe hellblond | Haselnuß Farbtiefe mittelblond | Leuchtendes Orangegelb Farbtiefe lichtblond |

### 4.3 Versuche mit Verbindungen der Formel (1) und Indol(in)derivaten

Zusätzlich wurden 3 Versuche mit 5-Hydroxy-benzo[b]furan-3-on (XVIII) und/oder 3,5-Dihydroxy-benzo[b]-thiophen (I) in Kombination mit 5,6-Dihydroxyindolin durchgeführt.

Dazu wurden je 100mg der o.a. Farbstoffe zusammen mit 50mg 5,6-Dihydroxyindolin-hydrobromid, 50mg Ammoniumsulfat sowie 100mg Natrosol® HR 250 mit 9g Wasser versetzt und der pH-Wert mit einer 25%igen Ammoniaklösung auf einen Wert von 10 eingestellt. Anschließend wurde mit Wasser auf 10g aufgefüllt.

In diese Anwendungszubereitungen wurde je eine Haarsträhne (Kerling naturweiß, 2g) für 30 Minuten bei Raumtemperatur getaucht, anschließend mit Wasser gespült und getrocknet.

**Tabelle III:**

| **Rohstoff** | **% Rezeptur-Nr.** | | |
|---|---|---|---|
| | **1** | **2** | **3** |
| 5-Hydroxy-benzo[b]furan-3-on | 1,0 | - | 1,0 |
| 3,5-Dihydroxy-benzo[b]-thiophen | - | 1,0 | 1,0 |
| 5,6-Dihydroxyindolin-hydrobromid | 0,5 | 0,5 | 0,5 |
| Ammoniumsulfat | 0,5 | 0,5 | 0,5 |
| Ammoniak, 25%ig | ad pH 10 | ad pH 10 | ad pH 10 |
| Wasser | ad 100 | ad 100 | ad 100 |
| Ausfärbung | mattes Gelb Farbtiefe lichtblond | mattes Gelb Farbtiefe hellblond | warmes Beige Farbtiefe hellblond |

## Patentansprüche

1. Mittel zur Färbung keratinischer Fasern, enthaltend in einem kosmetisch akzeptablen Medium als Farbstoffvorprodukt mindestens ein Derivat des Benzo[b]furan-3-ons und/oder mindestens ein Derivat des Benzo[b]thiophen-3-ons und/oder deren Keto-Enol-Tautomeren, **dadurch gekennzeichnet, dass** es als Derivat des Benzo[b]furan-3-ons und/oder des Benzo[b]thiophen-3-ons mindestens eine Verbindung der Formel (1) 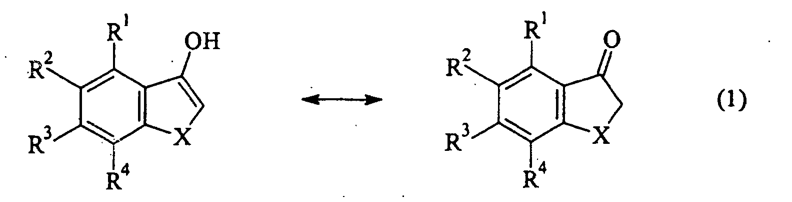 worin
X steht für Schwefel oder Sauerstoff und
die Reste R¹, R², R³ und R⁴ stehen unabhängig voneinander für
- Wasserstoff,
- eine C₁₋₄-Alkylgruppe, die gegebenenfalls mit
- einer oder mehreren Hydroxygruppe(n)
- einer gegebenenfalls mit einer C₁₋₄-Alkylgruppe veresterten Carboxygruppe oder
- einer Gruppe -SO₃R⁵, wobei R⁵ steht für Wasserstoff oder eine C₁₋₄-Alkylgruppe
substituiert sein kann,
- eine gegebenenfalls substituierte Amino- C₁₋₄-alkylgruppe,
- eine gegebenenfalls substituierte Amino-C₁₋₄-alkylaminogruppe,
- eine perfluorierte C₁₋₄-Alkylgruppe,
- eine Cyanogruppe,
- eine Allylgruppe,
- eine Vinylgruppe,
- ein Halogenatom,
- eine Gruppe -SO₃R⁵ oder
- eine Gruppe -OR⁶, wobei R⁶ steht für
- Wasserstoff,
- eine C₁₋₄-Alkylgruppe, die gegebenenfalls mit
- einer oder mehreren Hydroxygruppe(n),
- einer gegebenenfalls mit einer C₁₋₄-Alkylgruppe veresterten Carboxygruppe oder
- einer Gruppe -SO₃R⁵
substituiert sein kann,
- eine perfluorierte C₁₋₄-Alkylgruppe,
enthält.

2. Mittel nach Anspruch 1, **dadurch gekennzeichnet, dass** R¹ für Wasserstoff steht.

3. Mittel nach einem der Ansprüche 1 oder 2, **dadurch gekennzeichnet, dass** R⁴ für Wasserstoff steht.

4. Mittel nach einem der Ansprüche 1 bis 3, **dadurch gekennzeichnet, dass** R² für einen Substituenten der Formel -OR⁶ steht.

5. Mittel nach einem der Ansprüche 1 bis 4, **dadurch gekennzeichnet, dass** X für ein Sauerstoffatom steht.

6. Mittel nach Anspruch 5, **dadurch gekennzeichnet, dass** das Benzo[b]furan-3-onderivat der Formel (1) ausgewählt ist aus 5-Carboxymethoxy-benzo[b]furan-3-on, 5-Hydroxy- benzo[b]furan-3-on oder 5-(2'-Hydroxyethoxy)-benzo[b]furan-3-on.

7. Mittel nach einem der Ansprüche 1 bis 6, **dadurch gekennzeichnet, dass** X steht für ein Schwefelatom.

8. Mittel nach Anspruch 7, **dadurch gekennzeichnet, dass** das Benzo[b]thiophen-3-on-derivat der Formel (1) ausgewählt ist aus 5-Hydroxy-benzo[b]thiophen-3-on, 5-Carboxymethoxy-benzo[b]thiophen-3-on, 5,6-Dihydroxy-benzo[b]thiophen-3-on oder 6-Hydroxy-5-methoxy-benzo[b]thiophen-3-on.

9. Mittel nach einem der Ansprüche 1 bis 8, **dadurch gekennzeichnet, dass** es weiterhin mindestens ein Oxidationsfarbstoffvorprodukt vom Entwickler- oder Kupplertyp enthält.

10. Mittel nach einem der Ansprüche 1 bis 9, **dadurch gekennzeichnet, dass** es weiterhin mindestens ein Farbstoffvorprodukt vom Indol- oder Indolintyp enthält.

11. Mittel nach einem der Ansprüche 1 bis 10, **dadurch gekennzeichnet, dass** es weiterhin mindestens einen direktziehenden Farbstoff enthält.

12. Verfahren zum Färben keratinischer Fasern, **dadurch gekennzeichnet, dass** ein Mittel gemäß Anspruch 1 auf die Fasern aufgetragen wird und nach einer Einwirkungszeit wieder abgespült wird.

13. Verwendung von Derivaten des Benzo[b]furan-3-ons, des Benzo[b]thiophen-3-ons und/oder deren Keto-Enol-Tautomeren gemäß Anspruch 1 zur Färbung keratinischer Fasern.

14. 5-Carboxymethoxy-benzo[b]furan-3-on.

15. 5-(2'-Hydroxyethoxy)-benzo[b]furan-3-on.

16. 5-Carboxymethoxy-benzo[b]thiophen-3-on.

17. 5,6-Dihydroxy-benzo[b]thiophen-3-on.

18. 6-Hydroxy-5-methoxy-benzo[b]thiophen-3-on.

## Claims

1. Composition for colouring keratinous fibres which contains as dye precursors in a cosmetically acceptable medium at least one derivative of benzo[b]furan-3-one and/or at least one derivative of benzo[b]thiophen-3-one and/or keto-enol tautomers thereof, **characterized in that** it contains as the benzo[b]furan-3-one and/or benzo[b]thiophen-3-one derivative at least one compound corresponding to formula (1): 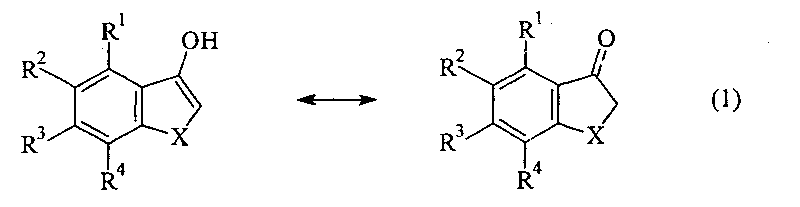 in which
X is sulfur or oxygen and
the substituents R¹, R³ and R⁴ independently of one another represent
- hydrogen,
- a C₁₋₄ alkyl group which may optionally be substituted by
- one or more hydroxy group(s)
- a carboxy group optionally esterified with a C₁₋₄ alkyl group or
- a group -SO₃R⁵, where R⁵ is hydrogen or a C₁₋₄ alkyl group,
- an optionally substituted amino-C₁₋₄-alkyl group,
- an optionally substituted amino-C₁₋₄-alkylamino group,
- a perfluorinated C₁₋₄ alkyl group,
- a cyano group,
- an allyl group,
- a vinyl group,
- a halogen atom
- a group -SO₃R⁵ or
- a group -OR⁶ where R⁶ is
- hydrogen,
- a C₁₋₄ alkyl group which may optionally be substituted by
- one or more hydroxy group(s)
- a carboxy group optionally esterified with a C₁₋₄ alkyl group or
- a group -SO₃R⁵,
- a perfluorinated C₁₋₄ alkyl group.

2. A composition as claimed in claim 1, **characterized in that** R¹ is hydrogen.

3. A composition as claimed in claim 1 or 2, **characterized in that** R⁴ is hydrogen.

4. A composition as claimed in any of claims 1 to 3, **characterized in that** R² is a substituent with the formula -OR⁶.

5. A composition as claimed in any of claims 1 to 4, **characterized in that** X is a sulfur atom.

6. A composition as claimed in claim 5, **characterized in that** the benzo[b]furan-3-one derivative corresponding to formula (I) is selected from 5-carboxymethoxybenzo[b]furan-3-one, 5-hydroxybenzo[b]furan-3-one or 5-(2'-hydroxyethoxy)- benzo[b]furan-3-one.

7. A composition as claimed in any of claims 1 to 6, **characterized in that** X is a sulfur atom.

8. A composition as claimed in claim 7, **characterized in that** the benzo[b]thiophen-3-one derivative corresponding to formula (I) is selected from 5-hydroxybenzo[b]thiophen-3-one, 5-carboxymethoxybenzo[b]thiophen-3-one, 5,6-dihydroxybenzo[b]thiophen-3-one or 6-hydroxy-5-methoxybenzo[b]thiophen-3-one.

9. A composition as claimed in any of claims 1 to 8, **characterized in that** it additionally contains at least one oxidation dye precursor of the primary intermediate and/or secondary intermediate type.

10. A composition as claimed in any of claims 1 to 9, **characterized in that** it additionally contains at least one dye precursor of the indole or indoline type.

11. A composition as claimed in any of claims 1 to 10, **characterized in that** it additionally contains at least one substantive dye.

12. A process for colouring keratinous fibres, **characterized in that** the composition claimed in claim 1 is applied to the fibres and, after a contact time, is rinsed off again.

13. The use of the derivatives of benzo[b]furan-3-one, benzo[b]thiophen-3-one and/or keto-enol tautomers thereof claimed in claim 1 for colouring keratinous fibres.

14. 5-Carboxymethoxybenzo[b]furan-3-one.

15. 5-(2'-Hydroxyethoxy)- benzo[b]furan-3-one.

16. 5-Carboxymethoxybenzo[b]thiophen-3-one.

17. 5,6-Dihydroxybenzo[b]thiophen-3-one.

18. 6-Hydroxy-5-methoxybenzo[b]thiophen-3-one.

## Revendications

1. Agent pour la teinture de fibres kératiniques, contenant dans un milieu cosmétiquement acceptable, comme précurseur de colorant, au moins un dérivé de la benzo[b]furann-3-one et/ou au moins un dérivé de la benzo[b]thiophén-3-one et/ou de leurs tautomères céto-énol, **caractérisé en ce qu'**il contient comme dérivé de la benzo[b]furann-3-one et/ou de la benzo[b]-thiophén-3-one au moins un composé de formule (1) 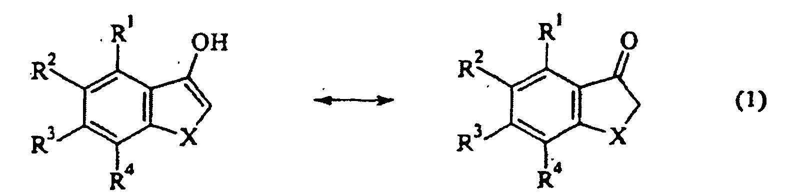 dans laquelle
X représente du soufre ou de l'oxygène et
les radicaux R¹, R², R³ et R⁴ représentent, indépendamment l'un de l'autre
- hydrogène,
- un groupe alkyle en C₁ à C₄, qui peut éventuellement être substitué par
- un ou plusieurs groupes hydroxy,
- un groupe carboxy éventuellement estérifié par un groupe alkyle en C₁ à C₄, ou
- un groupe -SO₃R⁵, où R⁵ représente hydrogène ou un groupe alkyle en C₁ à C₄,
- un groupe aminoalkyle en C₁ à C₄ éventuellement substitué,
- un groupe amino(alkyle en C₁ à C₄)amino éventuellement substitué,
- un groupe alkyle en C₁ à C₄, perfluoré,
- un groupe cyano,
- un groupe allyle,
- un groupe vinyle,
- un atome d'halogène
- un groupe -SO₃R⁵ ou
- un groupe -OR⁶, où R⁶ représente
- hydrogène
- un groupe alkyle en C₁ à C₄, qui peut le cas échéant être substitué par
- un ou plusieurs groupes hydroxy,
- un groupe carboxy éventuellement estérifié par un groupe alkyle en C₁ à C₄, ou
- un groupe -SO₃R⁵,
- un groupe alkyle perftuoré.

2. Agent selon la revendication 1, **caractérisé en ce que** R¹ représente hydrogène.

3. Agent selon l'une quelconque des revendications 1 ou 2, **caractérisé en ce que** R⁴ représente hydrogène.

4. Agent selon l'une quelconque des revendications 1 à 3, **caractérisé en ce que** R² représente un substituant de formule -OR⁶.

5. Agent selon l'une quelconque des revendications 1 à 4, **caractérisé en ce que** X représente un atome d'oxygène.

6. Agent selon la revendication 5, **caractérisé en ce que** le dérivé de benzo[b]furann-3-one de formule (1) est choisi parmi la 5-carboxyméthoxybenzo[b]furann-3-one, la 5-hydroxy-benzo[b]furann-3-one ou la 5-(2'-hydroxyéthoxy)benzo[b]furann-3-one.

7. Agent selon l'une quelconque des revendications 1 à 6, **caractérisé en ce que** X représente un atome de soufre.

8. Agent selon la revendication 7, **caractérisé en ce que** le dérivé de la benzo[b]thiophén-3-one de formule (1) est choisi parmi la 5-hydroxybenzo[b]thiophén-3-one, la 5-carboxyméthoxybenzo[b]thiophén-3-one, la 5,6-dihydroxybenzo[b]thiophén-3-one ou la 6-hydroxy-5-méthoxybenzo[b]thiophén-3-one.

9. Agent selon l'une quelconque des revendications 1 à 8, **caractérisé en ce qu'**il contient en outre au moins un précurseur d'un colorant d'oxydation du type agent de développement ou agent de couplage.

10. Agent selon l'une quelconque des revendications 1 à 9, **caractérisé en ce qu'**il contient en outre au moins un précurseur de colorant du type indole ou indoline.

11. Agent selon l'une quelconque des revendications 1 à 10, **caractérisé en ce qu'**il contient en outre au moins un colorant montant directement sur la fibre.

12. Procédé pour la teinture de fibres kératiniques, **caractérisé en ce qu'**on applique un agent selon la revendication 1 sur les fibres et il est à nouveau éliminé par rinçage après un temps d'action.

13. Utilisation de dérivés de la behzo[b]furann-3-one, de la benzo[b]thiophén-3-one et/ou de leurs tautomères céto-énol selon la revendication 1 pour la teinture de fibres kératiniques.

14. 5-Carboxyméthoxybenzo[b]furann-3-one.

15. 5-(2'-Hydroxyéthoxy)benzo[b]furann-3-one.

16. 5-Carboxyméthoxybenzo[b]thiophén-3-one.

17. 5,6-Dihydroxybenzo[b]thiophén-3-one.

18. 6-Hydroxy-5-méthoxybenzo[b]thiophén-3-one.
